# EUROPÄISCHE PATENTSCHRIFT

(11) **EP 1 368 111 B1**
(45) Veröffentlichungstag und Bekanntmachung des Hinweises auf die Patenterteilung: **10.10.2007**
(21) Anmeldenummer: 02719664.1
(22) Anmeldetag: 01.03.2002
(51) Int. Cl.: B01D 71/64, B01D 67/00

(54) **VERFAHREN ZUR HERSTELLUNG EINER TRÄGERMEMBRAN EINES BIOHYBRID-ORGANS AUSGEHEND VON EINER ASYMMETRISCH FUNKTIONALISIERTEN PORÖSEN POLYIMID-MEMBRAN SOWIE BIOHYBRID-ORGAN DIESE ENTHALTEND**
PROCESS FOR THE PRODUCTION OF A CARRIER MEMBRANE OF A BIOHYBRIDE ORGAN FROM A ASYMMETRICALLY FUNCTIONALIZED POROUS POLYIMIDE MEMBRANE AND BIOHYBRIDE ORGAN COMPRISING SAID MEMBRANE
PROCÉDÉ DE PRODUCTION D'UNE MEMBRANE SUPPORT D'UN ORGANE BIOHYDRIDE À PARTIR D'UNE MEMBRANE POLYIMIDE FONCTIONNALISÉE DE MANIERE ASYMÈTRIQUE POREUSE ET ORGANE BIOHYDRIDE LA COMPRENANT

(30) Priorität: 09.03.2001 DE 10111664
(43) Veröffentlichungstag der Anmeldung: 10.12.2003
(73) Patentinhaber: GKSS-Forschungszentrum Geesthacht GmbH, 21502 Geesthacht (DE)
(72) Erfinder: ALBRECHT, Wolfgang, 14513 Teltow (DE); GROTH, Thomas, 10439 Berlin (DE); PAUL, Dieter, 14532 Kleinmachnow (DE); SEIFERT, Barbara, 10249 Berlin (DE); FEY-LAMPRECHT, Frédérique, 85521 Ottobrunn (DE); GROSS, Ulrich, 14195 Berlin (DE)
(74) Vertreter: Seemann, Ralph
(86) Internationale Anmeldenummer: PCT/DE2002/000768
(87) Internationale Veröffentlichungsnummer: WO 2002/072249

(56) Entgegenhaltungen:
- EP-A- 0 401 005
- EP-A- 0 586 268
- DE-A- 4 117 501
- US-A- 4 853 127
- KEN-ICHI OKAMOTO ET AL: "SELECTIVE PERMEATION OF CARBON DIOXIDE THROUGH AMINE-MODIFIED POLYIMIDE MEMBRANES" CHEMISTRY LETTERS, CHEMICAL SOCIETY OF JAPAN. TOKYO, JP, April 1996 (1996-04), Seiten 613-614, XP002929041 ISSN: 0366-7022

## Beschreibung

Die Erfindung betrifft ein Verfahren zur Herstellung einer Trägermembran eines Biohybridorgans aus einer asymmetrisch funktionalisierten porösen Polyimid-Membran sowie ein Biohybridorgan.

Die extrakorporale Detoxifikation von Blut unter Verwendung von Membranen hat sich als Technik zur Therapie von Organversagen, beispielsweise der Niereninsuffizienz, bewährt und wird heute weltweit umfangreich angewandt. Bei diesem Entgiftungsvorgang permeieren entsprechend der Porengröße der Membran und der Molekülgröße der zu entfernenden Stoffe Toxine aus dem Blut des Patienten in eine Spüllösung (Dialysat). Dies wird hier als ein "passiver" Transport auf Basis von Konzentrationsunterschieden von Inhaltsstoffen in beiden Flüssigkeiten betrachtet.

In der natürlichen Niere erfolgt die Entfernung der Uremietoxine in erster Stufe ebenfalls auf Basis eines passiven Prozesses, bei dem der Primärharn entsteht. Im Gegensatz zur künstlichen Niere ist jedoch diesem passiven Ultrafiltrationsprozeß ein aktiver Prozeß nachgeschaltet, bei dem Inhaltsstoffe des Primärharnes und Wasser entgegen dem Konzentrationsgradienten vom Primärharn zurück ins Blut transportiert werden. Eine derartige Funktion (und auch weitere Funktionen) sind beim gegenwärtigen Entwicklungsstand der Membran-Trenntechnik nur mittels vitaler und gewebartig organisierter Zellschichten realisierbar. Entsprechendes gilt sinngemäß auch für andere Organe. Mittels Porenmembranen lassen sich derzeit lediglich eine passive Filtrationsfunktion und bestenfalls eine sorptive Funktion der für die Entgiftung zuständigen Organe (Nieren und Leber) realisieren und folglich therapeutisch nutzbar machen.

Es wurden bereits zahlreiche Versuche durchgeführt, um den aktiven Transport vitaler Zellen gezielt zur Unterstützung von Dysfunktionen kranker Organe anzuwenden. Bis heute sind die hierbei erzielten Wirkungen therapeutisch kaum nutzbar. Dies liegt daran, daß einerseits die Vitalität der Zellen nicht über den notwendigen Zeitraum aufrechterhalten werden kann und andererseits die Funktionalität der bisher realisierten Zellverbände bei weitem nicht die Funktionalität des entsprechendes natürlichen Gewebes erreicht.

Ein Hauptgrund für diese unbefriedigende Funktionalität liegt darin, daß der Membranträger hinsichtlich der Membranfunktion nur passiv und nicht aktiv wirkt. Für eine optimale Funktionalität der Zellen müßte ein Membranträger jedoch über zahlreiche Eigenschaften gleichzeitig verfügen, um erfolgreich eingesetzt werden zu können.

Es wurden bereits zahlreiche Versuche unternommen, geeignete Membranträger mit einem für den speziellen Anwendungsfall erforderlichen Eigenschaftsprofil zu entwickeln. Prinzipiell können diese bekannten Membranträger in drei unterschiedliche Grundtypen differenziert werden, die sich insbesondere in den Membran- und/oder Quellungseigenschaften unterscheiden, nämlich hochquellende Gelmembranen mit Membranfunktion, praktisch nicht-quellende Porenmembranen mit Membranfunktion und 3D-Matrix-Materialien ohne Trennfunktion.

Letztere 3D-Matrix-Materialien ohne Trennfunktion, die als Zellträger dienen und aus unterschiedlichen Polymeren mit grobporiger Porenstruktur hergestellt sind, werden beispielsweise in der GB-A 2 187 447 beschrieben. Das Porensystem ist dabei derart, daß die Zellen im Porensystem verankert werden, dort poliferieren und gewebeähnliche Strukturen bilden. Auch die Integration einer Begasungs-Hohlmembran in das Trägersystem bzw. den Biohybridreaktor ist bereits bekannt (W0 97/12960). Die Zellkultur kommt bei dieser Art von Trägermaterial mit Blut oder dessen Bestandteilen ohne Immunisolation in Kontakt. Mit derart strukturierten 3D-Trägern ist somit keine Immunisolation möglich. Dies würde bei einem Einsatz im humanmedizinischen Bereich die Verwendung von Patientenzellen erforderlich machen, die normalerweise nicht zugänglich sind. Daher erfüllen derartige Träger nicht das Anforderungsprofil für eine Bioreaktor-Membran.

Einen weiteren Typ von Bioreaktor-Membran stellen quellungsstabile Porenmembranen sowie quellungsfähige Cellulosepolymere (WO 95/21911) dar. Eine Vielzahl von membranbildenden Polymeren wurden für die Anwendung in Bioreaktoren vorgeschlagen. Eine typische Bioreaktor-Membran aus Poly(vinylchlorid-acrylnitril)-Copolymeren ist in Diabetes 35 (1986), 625 beschrieben. Diese Membran soll leicht unter Erhalt von immunisolierenden Eigenschaften herzustellen sein. Sie stellt die am häufigsten in Bioreaktoren eingesetzte Membran dar und besitzt einen asymmetrischen Aufbau mit einer Vielzahl von Radialkapillaren, die sich praktisch über den gesamten Membranquerschnitt erstrecken. Diese bekannte Bioreaktor-Membran besitzt insbesondere den Nachteil, daß sie mechanisch sehr instabil ist. Bereits eine geringe Verletzung der immunisolierenden Membranoberflächen zerstört diese Funktion der Membran.

Weitere Bioreaktor-Membranen wurden auf Basis von Polyacrylnitril-Polymeren und anderer Acrylnitril-Polymere realisiert. Dabei wurde die Polyacrylnitril-Membran durch Pfropfung von Polyethylenglykol-Derivaten an die zuvor aktivierte PAN-Membran dauerhaft hydrophiliert und als Folge einer geringen Proteinadsorption hämokompatibel gestaltet (US-A 5720969). Diese Modifizierung erfordert zahlreiche, komplizierte Modifizierungsstufen, die an der formierten Membran durchgeführt werden müssen. Zudem ist auch bei diesen Membranen die immunisolierende Membranschicht leicht verletzbar. Außerdem entstehen bei der Polymermodifizierung hochquellende Polymerprodukte, die ohne zusätzliche Vernetzung die mechanischen Anforderungen an eine Biohybridreaktor-Membran nicht erfüllen.

Zur Vermeidung des Nachteils einer leichten Verletzbarkeit der immunisolierenden Schicht der Membran wird in der WO 96/40871 die Verwendung einer Polysulfon-Membran mit feinporiger Schaumstruktur für Bioreaktor-Membranen-Anwendungen beschrieben. In der US-A 5837234 wird die Immobilisierung von Zellen auf Polysulfon mit asymmetrischer Morphologie und Schaumstruktur beschrieben. Nachteilig ist jedoch, daß Membranen aus Polysulfon nur dann als hämokompatibel zu bezeichnen sind, wenn sie nur kurze Zeit mit Blut in Kontakt kommen. Diese Membranen neigen insbesondere als Folge der Proteinadsorption stark zum Fouling, wodurch sich deren Trenneigenschaften verändern.

Bekanntlich kann die Proteinadsorption und damit die Hämokompatibilität einer Membran durch Hydrophilierung/Passivierung z. B. mit Polyethylenglykolderivaten oder Acrylsäurederivaten, verringert bzw. verbessert werden, wenn diese Stoffe adsorptiv, ionisch oder insbesondere kovalent an der Membranoberfläche fixiert werden. Ebenso kann durch Modifizierung/Beschichtung mit entsprechenden Substanzen die Gewebeverträglichkeit und das Adhäsionsverfahren von Zellen beeinflußt werden (D.A.Stenger u.a.: Brain Res. 630 (1993), 136). Zur Verbesserung der Hämokompatibilität ist eine temporäre Hydrophilisierung mit anderen hydrophilen Stoffen bei Polysulfon und anderen, in Biohybridreaktoren eingesetzten Polymer-Membranen bekannt (US-A 4 675 213 und US-A 4 794 002). Eine Langzeitstabilität der hydrophilen Eigenschaften derartig modifizierter Membranen kann mittels dieser Vorgehensweise nicht, sondern nur mittels kovalenter Bindung des Modifikators an die Membran gewährleistet werden.

Eine naß-chemische Funktionalisierung einer Polysulfon- Membran zur dauerhaften Verbesserung der Eigenschaften mittels kovalenter chemischer Kopplung des Modifikators stößt auf schwerwiegende Probleme, da Polysulfone chemisch relativ beständig sind und a priori kaum nutzbare funktionelle Gruppen aufweisen, die für eine kovalente Kopplung des Modifikators an die Membran verwendet werden können. Auch eine asymmetrische Funktionalisierung erscheint bei Verwendung von Polysulfon als Membranpolymer praktisch nicht möglich.

Naß-chemische Modifizierungen von Polyamid-Membranen zur Veränderung des Adsorptionsverhaltens sind in J. Membrane Sci 129 (1997), 31, beschrieben. Bei diesem Verfahrensprinzip werden Amin-Endgruppen des Polyamids genutzt, um eine celluloseartige Coating-Schicht kovalent an Membranträger zu binden. Die funktionellen Gruppen der Coating-Schicht werden für eine zweite Kopplung des Target-Liganden der Funktionalisierung benutzt. Diese Funktionalisierungstechnik ist aufwendig und teuer.

Weiterhin ist in EP-A-0 401 005 ein Verfahren zur chemischen Modifizierung einer polymeren Gastrennmembran beschrieben, wobei Ammoniak oder eine Aminoverbindung wenigstens einer Seite der Membran zugeführt wird. Ferner wird die Membran aus Polyimid gebildet. Gemäß der technischen Lehre werden Mono-, Di- und multivalente Amine als Modifikatoren der Membran eingesetzt, die ausschließlich dem Ziel dienen, das Membranpolymer zu vernetzen und unlöslich mit üblichen Polyimid-Mitteln zu machen. Bei der Gastrennmembran werden dichte, d.h. unporöse Filme verwendet, so dass derartige Membranen an sich nicht als Trägermembran eines Biohybridorgans einsetzbar sind, da die Gewährleistung des Stoffaustausches bei Biohybridorganen nicht gegeben ist.

Darüber hinaus ist aus EP-A-0 586 268 ein Material zur Entfernung von Viren aus einer proteinhaltigen Lösung bekannt, das ein Basismaterial, eine auf einer Oberfläche des Basismaterials eingeführte Oberflächenpfropfkette und eine auf eine Oberfläche des Basismaterials durch die Oberflächenpfropfkette immobilisierte Polyaminverbindung enthält. Um einen Blutfilter auszubilden, wird das die pathogenen Substanzen selektiv entfernende Material in Form einer porösen Membran bereitgestellt.

Weiterhin ist aus DE-A-41 17 501 ein Verfahren zur Herstellung einer aminmodifizierten Polyimid-Membran bekannt, wobei in einer Gießlösung, die Polyimide enthält, lösliche organische mehrfunktionelle Aminverbindungen mit mindestens zwei primären Aminogruppen pro Molekül versetzt werden. Dieser Stand der Technik betrifft somit ein Verfahren zur Modifizierung von Polyimiden in einer Gießlösung bzw. die Umsetzung von Polyimiden in gelöster Form bzw. in Lösung und somit eine homogene Reaktion.

Aufgabe der vorliegenden Erfindung ist es, eine Trägermembran bereitzustellen, die in Biohybridreaktoren eingesetzt werden kann, ökonomisch herstellbar ist, entsprechend dem Einsatzziel einerseits permeabel genug, jedoch immunisolierend und andererseits sowohl hämo- als auch gewebeverträglich ist sowie ein gezielt einstellbares Proteinadsorptionsverhalten aufweist.

Gelöst wird diese Aufgabe durch eine Membran gemäß der Lehre der Ansprüche.

Erfindungsgemäß wird somit Polyimid als membranbildendes Polymer zur Membranherstellung eingesetzt und auf per se bekannte Weise zu einer Membran verformt. Diese Membran besitzt vorzugsweise eine schaumartige und daher radialkapillarenfreie Struktur.

Diese auf per se bekannte Weise hergestellte und als Ausgangsmaterial eingesetzt Polyimid-Membran wird von allen nicht-membranbildenden Bestandteilen, außer Wasser, befreit, insbesondere durch Waschen. Die so formierte Membran wird dann in feuchter Form oder nach Trocknung derart funktionalisiert, daß
a) eine Oberfläche der Polyimid-Membran mit einer wäßrigen Funktionalisierungslösung für einen Zeitraum von 1 sec. bis 1 h in Kontakt gebracht wird,
b) die Funktionalisierungslösung mindestens einen darin zumindest teilweise gelösten Modifikator enthält, der eine Aminogruppe zur kovalenten Kopplung des Modifikators an das Polyimid sowie zusätzlich mindestens eine weitere funktionelle Gruppe pro Molekül aufweist, wobei die weitere funktionelle Gruppe derart ausgewählt ist, daß sie nach der kovalenten Kopplung des Modifikators an das Polyimid und somit nach Funktionalisierung der Polyimid-Membran in freier Form vorliegt, wobei sie entsprechend dem Funktionalisierungsziel insbesondere eine hämokompatible und/oder gewebekompatible Wirkung aufweist, und
c) die Polyimid-Membran während oder nach dem in Kontakt bringen mit der Funktionalisierungslösung auf eine erhöhte Temperatur gebracht wird und anschließend gereinigt und getrocknet wird.

Die erfindungsgemäße Polyimid-Membran ist vorzugsweise dadurch erhältlich, daß sie für 5 sec. bis 10 min. mit der Funktionalisierungslösung in Kontakt gebracht wird. Weiterhin wird die Polyimid-Membran während des Kontaktes mit der Funktionalisierungslösung und/oder danach vorzugsweise 1 sec. bis 1 h und insbesondere 5 sec. bis 10 min. auf eine Temperatur von 50 - 100°C und insbesondere von 70 - 90°C erhitzt. Als Funktionalisierungslösung wird vorzugsweise eine wäßrige Lösung eingesetzt. Ferner wird die Polyimid-Membran vorzugsweise zum Reinigen in an sich bekannter Weise durch Waschen, Extrahieren etc. mit Wasser behandelt.

Die Gesamtkonzentration des Modifikators oder der Modifikatoren in der Funktionalisierungslösung beträgt vorzugsweise 0,1 bis 20 Gew.-% und weiterhin bevorzugt 1 bis 10 Gew.-%.

Bei der erfindungsgemäßen Membran wird somit eine Trägermembran aus einem einem bekannten, membranbildenden Polymer entsprechenden Trennprofil derart asymmetrisch und kovalent mit chemischen Funktionen modifiziert, daß sich auf der jeweiligen Membranoberfläche zumindest eine chemische Funktion mit der höchsten Konzentration befindet, wobei sich deren Gehalt über den Membranquerschnitt derart vermindert, daß an der gegenüberliegenden Membranoberfläche der geringste Gehalt an dieser Funktion fixiert ist, hier jedoch der höchste Gehalt der anderen Funktion nachweisbar ist. Erfindungsgemäß wird somit eine Biohybridreaktor-Membran bereitgestellt, die neben dem durch die Herstellungsparameter einstellbaren Trennprofil 1 bei optimaler Versorgung der Zellschicht immunisolierende Eigenschaften besitzen kann. Insbesondere kann die mit Blut, Plasma, Medium usw. kontaktierte Membranoberfläche hämokompatibel sein. Zudem kann die mit den Zellen kontaktierter Membranoberfläche gewebekompatibel gestaltet sein. Vorzugsweise ist die erfindungsgemäße Membran auch mit Dampf sterilisierbar.

Nachdem aus der funktionalisierten Membran in an sich bekannter Weise nicht gebundenen die Modifikatoren und/oder Hilfsstoffe aus der kontaktierenden Funktionalisierungslösung durch Waschen, Extrahieren oder ähnlichem entfernt wurden, kann die funktionalisierte Membran zudem mit an sich bekannten porenerhaltenden Substanzen imprägniert werden, bevor sie getrocknet wird. Erforderlichenfalls kann in einer zusätzlichen Nachbehandlungsstufe die funktionalisierte Membran mit weiteren, an sich bekannten Behandlungslösungen kontaktiert werden, um beispielsweise eine in Salzform vorliegende Funktion des Modifikator-Moleküls in die Säureform zu überführen oder eine neutrale Funktion, z. B. durch Quartenierung in eine ionische Form zu überführen.

Nach der Funktionalisierungsbehandlung steht eine entsprechend der Wahl des Modifikators oder der Modifikatoren funktionalisierte, im allgemeinen poröse Polyimid-Membran mit einem hohen Gehalt an funktionellen Gruppen auf zumindest einer Membranoberfläche zur Verfügung, deren gezielt einstellbare, je nach Reaktionsführung in hoher Zahl frei verfügbaren funktionellen Gruppen asymmetrisch über dem Membranquerschnitt verteilt sind. Bei der erfindungsgemäßen naß-chemisch asymmetrisch funktionalisierten Polyimid-Membran befinden sich zumindest zwei chemisch unterschiedliche Funktionen mit ihrer höchsten Funktionalität auf jeweils einer der beiden Membranenoberflächen; die Funktionalität nimmt, ausgehend von diesem Ort, mit zunehmender Entfernung zur anderen Membranoberfläche durch den Membranquerschnitt ab.

Ein analoger Funktionalisierungsschritt kann mit einer zweiten Funktionalisierungslösung anderer Zusammensetzung an der anderen Membranoberfläche durchgeführt werden. In diesem Falle werden dann die funktionell asymmetrisch modifizierte Membranen erhalten, bei denen sich der jeweils höchste Gehalt an Modifikator(en) auf der jeweiligen Membranoberfläche befindet(en), die mit der Funktionalisierungslösung in Kontakt war. Derart hergestellte Membranen entsprechender Trennwirkung, welche die geforderte Immunisolation im Bioreaktor gewährleisten, besitzen bei entsprechender Modifikatorauswahl sowohl eine hämokompatible als auch eine gewebekompatible Oberfläche und bieten damit beste Voraussetzungen den Anforderungen an einer Trägermembran in Biohybridorganen optimal gerecht zu werden.

Gegenstand der Erfindung ist daher auch die Verwendung der erfindungsgemäß naß-chemisch erhältlichen Polyimid-Membran in Biohybridsystemen, bei denen es sich um ein Hybrid aus "passiver" Membranfunktion und "aktiver" Zellfunktion handelt. Derartige Systeme mit einer erfindungsgemäß funktionalisierten Membran als Trägermembran können zur Unterstützung bzw. zum temporären Ersatz erkrankter Organe eingesetzt werden.

Die erfindungsgemäß eingesetzten Modifikatoren besitzen aus chemischer Sicht zumindest eine Amin-Gruppe, die primärer oder sekundärer Natur ist, um die erfindungsgemäße kovalente Kopplung des Modifikators oder der Modifikatoren an die als Ausgangsmaterial eingesetzte Polyimid-Membran zu realisieren. Ferner ist zumindest eine weitere Funktion vorhanden, die für die jeweilige Biokompatibilität der zu modifizierenden Membranoberfläche verantwortlich zeichnet.

Als Modifikatoren, die zu einer Verbesserung der Bioverträglichkeit bezüglich der Blutverträglichkeit führen, werden vorzugsweise Substanzen eingesetzt, die neben der Amin-Funktion chemische Gruppen wie beispielsweise Hydroxyl- und/oder Carboxyl- und/oder SulfonsäureGruppen besitzen und die Membranoberfläche hydrophilisieren und damit zu einer Passivierung der Membranoberfläche führen, wie beispielsweise funktionalisierte Polyethylenglykole und/oder Substanzen mit Antikoagulanzwirkung wie Heparin, Heparansulfat, Dextransulfate und Hirudin. Vorzugsweise werden für eine hämokompatible Funktionalisierung aminierte Polyethylenglykole unterschiedlicher Kettenlänge als Modifikatoren eingesetzt. Hinsichtlich der Auswahl der chemischen Funktion, die neben der erfindungsgemäßen notwendigen Aminfunktion eine optimale Gewebeverträglichkeit ermöglichen, müssen die spezifischen Anforderungen der für die Besiedlung vorgesehenen Zellen berücksichtigt werden. Die erforderliche chemische Funktion muß dabei experimentell ermittelt werden.

Es hat sich anhand von Zellbesiedlungsuntersuchungen jedoch als vorteilhaft erwiesen, die Oberflächen moderat hydrophil und/oder mit Hydroxyl-, und/oder Amin-Gruppen und weiteren Funktionen zu funktionalisieren. Es ist darüber hinaus möglich, zellspezifische Liganden, wie beispielsweise Bindungssequenzen (Polypeptide) von bestimmten Matrixproteinen oder Wachstumsfaktoren direkt oder über Spacermoleküle kovalent zu koppeln. Diesem Anforderungsprofil entsprechende Modifikatoren sind dem Fachmann bekannt und verfügbar.

Für den Fall, daß aminische Funktionen zur entsprechenden Gewebeverträglichkeit führen, werden vorzugsweise Diamine und Polyamine wie. Polyethylenimin oder bifunktionale Polyethylenglykole unterschiedlicher Kettenlänge als Modifikatoren eingesetzt.

Bei den Funktionalisierungslösungen, mit der die Membran kontaktiert wird, werden vorzugsweise wäßrige Medien eingesetzt, in denen die Modifikatoren zumindest teilweise und vorzugsweise vollständig löslich sind. Insbesondere bei der Funktionalisierung einer trockenen Membran in Form von Nachbehandlungsprozessen hat es sich zur Verbesserung der Benetzbarkeit der Membran als vorteilhaft erwiesen, dem wäßrigen Behandlungsmedium Anteile an mit Wasser vollständig mischbaren, kurzkettigen mono- oder mehrfunktionellen Alkoholen zuzusetzen und dieses Lösemittelgemisch als Lösemittel für den Modifikator oder den Modifikatoren einzusetzen. Der Anteil dieser Alkohole beträgt vorzugsweise 10 - 90 Gew.-% und weiterhin bevorzugt 20 - 50 Gew.-% in der Funktionalisierungslösung.

Die in den vorliegenden Unterlagen benutzten Begriffe "Polyimid" und "Polyimid-Membranen" bezeichnen neben dem reinen Polyimiden auch solche Stoffklassen wie Poly-(amid-imide), Poly(ester-imide), Poly(ether-imide) usw. sowie Polymergemische (Polymerblends), die einen Anteil aus den genannten Polymerklassen enthalten. Erfindungsgemäß ist es lediglich erforderlich, daß zumindest ein Anteil des membranbildenden Polymers oder der membranbildenden Polymere in der Polymerkette Imid-Gruppen enthalten. Folglich kann jedes diese Imid-Funktionen enthaltende Polymer bzw. jede Imid-Gruppen enthaltende Membran erfindungsgemäß funktionalisiert werden. Vorzugsweise werden jedoch reine Polyimid-Membranen eingesetzt.

Bei den erfindungsgemäß erhältlichen Membranen kann es sich sowohl um funktionalisierte Hohlfaden- und Schlauchmembranen als auch um funktionalisierte ebenflächige Membranen handeln.

Wie oben bereits dargelegt, kann man die als Ausgangsmaterial eingesetzte Polyimid-Membran von den beiden Oberflächen her unterschiedlich funktionalisieren. Bei geeigneter Wahl der funktionellen Gruppen der Modifikatoren erhält man im Ergebnis eine Membran, deren eine Membranoberfläche anders funktionalisiert ist als die andere Membranoberfläche. Vorzugsweise ist eine Membranoberfläche hämokompatibel und die andere Membranoberfläche gewebekompatibel funktionalisiert. Da diese Funktionalisierungsreaktionen zu einer kovalenten Bindung der Modifikatormoleküle an die als Ausgangsmaterial eingesetzte Polyimid-Membran führen, gewährleistet die erfindungsgemäß funktionalisierte Membran eine Langzeitstabilität des Funktionalisierungseffektes. Die Porenstruktur der Membran kann durch Parameter der Membranherstellung gezielt derart beeinflußt werden, daß ein Trennprofil eingestellt wird, daß das Immunsystem eines Patienten vor Reaktionen der allogenen bzw. xenogenen Zellen bei gleichzeitig ausreichender Versorgung des Zellkompartiments isoliert.

Gegenstand der Erfindung ist auch ein Verfahren gemäß der Lehre des unabhängigen Verfahrensanspruches. Mit diesem Verfahren ist es möglich, erfindungsgemäß funktionalisierte Membranen auf sehr ökonomische Art und Weise herzustellen.

Die Erfindung wird im folgenden anhand der nachstehenden Beispiele näher erläutert. Dabei wurden Polyetherimidmembranen als Ausgangsmaterial für die erfindungsgemäß funktionalisierten Polyimid-Membranen eingesetzt. Bei dem Polyetherimid handelte es sich um Ultem 1000 (General Electric), aus dem in bekannter Weise ebenflächige Membranen formiert wurden. Die Funktionalisierung wird dabei beispielhaft anhand der Modifizierung mit aminierten Polyethylenglycol bzw. Heparin-Na-Salz zur Erzeugung einer hämokompatiblen Oberfläche und mit Tris(hydroxymehtyl)-aminomethan bzw. N,N-Dimethylethylendiamin (asymmetrisch) zur Erzeugung einer gewebeverträglichen Oberfläche beschrieben. Zur Charakterisierung der Bioverträglichkeit und Morphologie der Zellschichten der in den Beispielen eingesetzten Membranen wurden die folgenden, dem Fachmann bekannten Techniken herangezogen:
1. Bestimmung des Glycoproteins P-Selektin mittels ELISA zur Ermittlung der Thrombozytenadhäsion und -aktivierung beim Kontakt mit den Membranen.
2. Plasmakallikrein-Assay als Maß für die Kontaktivierung von Blut im Kontakt mit den Membranen.
3. Nicht-aktivierte partielle Thromboplastinzeit (nAPTT) als Kenngröße zur Charakterisierung der Aktivierung der Blutgerinnung.
4. Bb-Fragment-ELISA zur Charakterisierung der Komplementaktivierung des Blutes beim Membrankontakt.
5. MTT- und LDH-Test zur Charakterisierung der Zellproliferation und -vitalität.
6. Albumin-ELISA zur Bewertung der Zellfunktionalität.
7. REM-Mikroskopie von Zellschichten.

Hierzu wurden die Zellschichten auf den Membranen mit 3 Gew.-% Glutaraldehyd-Lösung fixiert, in einer aufsteigenden Ethanolreihe entwässert und einer Kritischer-Punkt-Trocknung unterzogen.

### Beispiel 1

Eine PEI-Flachmembran (Hersteller: GKSS-Forschungszentrum Geesthacht) mit Ultrafiltrationseigenschaften wurde im trockenen Zustand, montiert auf eine Metalltrommel, von der Aktivschichtseite her mit einer Modifikator-Lösung, bestehend aus 2 Gew.-% Tris(hydroxymethyl)-aminomethan, 49 Gew.-% 1-Propanol und 49 Gew.-% Wasser, bei 70°C für 1 min. bzw. 10 min. unter Drehen des Metallzylinders kontaktiert und anschließend intensiv mit Wasser gewaschen. Die erhaltenen Membranen wurde im Beispiel 6 für Zellkulturuntersuchungen eingesetzt.

### Beispiel 2

Entsprechend dem Beispiel 1 wurden weitere PEI-Flachmembranen mit den Unterschieden funktionalisiert, daß
* einerseits aminiertes Polyethylenglykol bzw. Heparin-Na-Salz anstelle von Tris(hydroxymethyl)-aminomethan verwendet wurde und
* andererseits die zu funktionalisierenden Membranen in die Modifikator-Lösung eingelegt wurden.

Die so funktionalisierten Membranen wurden zur Charakterisierung der Blutverträglichkeit eingesetzt; die Ergebnisse der Untersuchungen sind in den Beispielen 4 und 5 wiedergegeben.

### Beispiel 3

Aus trockenen PEI-Hohlmembranen mit Ultrafiltrationseigenschaften (eigene Laborherstellung) wurden Rohrbündel hergestellt, in ein Modulgehäuse aus Glas eingeführt und an beiden Enden mit Silikonkautschuk flüssigkeitsdicht eingebettet. Das äußere Kompartiment des Modules wurde mit einer Modifikator-Lösung, bestehend aus 2 Gew.-% N,N-Dimethylethylendiamin, 49 Gew.-% 1-Propanol und 49 Gew.-% Wasser gefüllt; das innere Kompartiment wurde verschlossen. Anschließend wurde der so vorbereitete, gefüllte Modul in einem Trockenschrank 6 min. bei 70°C behandelt. Nach Abkühlen wurden beide Kompartimente des Modules und die Wand der Hohlmembranen intensiv mit Wasser gespült. Die so funktionalisierten Hohlmembranen wurden nach Trocknung bei Raumtemperatur dem Modul entnommen und - in einen Bioreaktor mit Faser-in-Faser-Design eingebaut - für Zellkulturuntersuchungen eingesetzt. Die Ergebnisse dieser Untersuchungen sind im Beispiel 7 beschrieben.

### Beispiel 4

Membranen aus PEI wurden mit einer 2 % Aminomethoxypolyethylenglykol enthaltenden Lösung, gelöst in n-Propanol/Wasser-Gemisch (Mischungsverhältnis: 1:1) für 1 bzw. 10 min. bei 70°C behandelt und anschließend intensiv mit Wasser gewaschen. Der Einfluß dieser Modifizierung auf die Blutkontakteigenschaften der Membranen wurde mittels P-Selektin- und Plasmakallikrein-Assay eingeschätzt. Die Ergebnis dieser Untersuchungen sind in Tabelle 1 zusammengestellt.

**Tabelle 1:**

| P-Selektin- und Plasmakallikrein-Assay | | | | |
|---|---|---|---|---|
| Membran | P-Selektin (0.D./492 nm) | | Plasmakallikrein-Aktivierung (0.D./405 nm) | |
| | Mittelwert | Standardabweichung | 30 min Kontakt | 60 min Kontakt |
| PEI | 0,728 | 0,474 | 0,050 | 0,059 |
| PEI-PEG-1 | 0,346 | 0,245 | 0,038 | 0,045 |
| PEI-PEG-10 | 0,675 | 0,077 | -- | -- |

Die PEG-Modifizierung von PEI bewirkt eine Passivierung der Oberfläche - weniger Thrombozyten lagern sich an und zeigen eine geringere Expression von P-Selektin, einem Marker der Thrombozytenaktivierung, auf ihrer Oberfläche. Besonders die kurzzeitige Modifizierung (1 min.) reduziert die Thrombozytenadhäsion und -aktivierung um mehr als 50%. Auch die Aktivierung von Plasmakallikrein, einer Protease, die bei der Blutgerinnung aus Präkallikrein entsteht und u. a. den Faktor XII (Hagemann-Faktor) aktiviert, ist auf PEI-Membranen, die 1 min. mit Aminomethoxy-PEG modifiziert wurden, um 25 % verringert. PEG-modifizierte PEI-Membranen besitzen somit eine inertere und damit hämokompatiblere Oberfläche als unmodifizierte PEI-Membranen.

### Beispiel 5

Membranen aus PEI wurden mit einer 2 % Heparin-Na-Salz enthaltenden Lösung, gelöst in n-Propanol/Wasser-Gemisch (Mischungsverhältnis: 1:1) für 1 bzw. 10 min. bei 70°C behandelt und anschließend intensiv mit Wasser gewaschen. Der Einfluß dieser Modifizierung auf die Blutkontakteigenschaften der Membranen wurde mittels nichtaktivierten partiellen Thromboplastinzeit (nAPTT) und Bb-Fragment-ELISA eingeschätzt. Die Ergebnisse dieser Untersuchungen sind in Tabelle 2 zusammengestellt.

**Tabelle 2:**

| Nicht-aktivierte partielle Thromboplastinzeit (nAPTT, Kontrolle = Plasma ohne Materialkontakt) und Komplementaktivierung | | | |
|---|---|---|---|
| Membran | nAPTT (% der Kontrolle) | Komplementaktivierung Bb-Fragment (µg/ml) | |
| | | Mittelwert | Standardabweichung |
| PEI | 89 | 6,79 | 1,43 |
| PEI-Hep-1 | 102 | 2,54 | 1,10 |
| PEI-Hep-10 | 110 | 5,04 | 0,78 |

Die Heparin-Modifizierung von PEI bewirkt eine geringere Aktivierung der Blutgerinnung, ersichtlich aus den Werten der nAPTT, die den inneren Koagulationspfad (Faktoren XII, XI, IX, VIII, X, V, II und I) charakterisiert. Nach 30 min. Kontakt von plättchenarmen humanen Blutplasma mit den Membranen ist die nAPTT im Gegensatz zum unmodifizierten PEI für Heparin-modifizierte PEI-Membranen gegenüber der Kontrolle (Plasma ohne Materialkontakt) sogar verlängert. Der Gehalt des Fragments Bb im Blutplasma weist für die modifizierten PEI-Membranen geringere Werte auf, was auf eine geringere Aktivierung des Komplementsystems schließen läßt. Heparin-modifizierte PEI-Membranen besitzen eine weniger gerinnungs- und Komplementsystem-aktivierende und somit hämokompatiblere Oberfläche als unmodifizierte PEI-Membranen.

### Beispiel 6

Membranen aus PEI wurden mit einer 2 Gew.-% Tris(hydroxymethyl)-aminomethan enthaltenden Lösung, gelöst in einem n-Propanol/Wasser-Gemisch (Mischungsverhältnis: 1:1) für 1 bzw. 10 min. bei 70°C behandelt und anschließend intensiv mit Wasser gewaschen. Der Einfluß dieser Modifizierung auf die Gewebeverträglichkeit wurde im direkten Kontakt der Membran mit 3T3-Fibroblasten untersucht.

**Tabelle 3:**

| Zelladhäsion und -proliferation von 3T3-Zellen, charakterisiert mittels MTT-Test | | | | |
|---|---|---|---|---|
| Membran | MTT-Test (0.D./562 nm) | | | |
| | nach 2 d Kultivierung | | nach 4 d Kultivierung | |
| | Mittelwert | Standardabweichung | Mittelwert | Standardabweichung |
| PEI | 0,237 | 0,088 | 0,400 | 0,019 |
| PEI-0H-1 | 0,330 | 0,078 | 0,614 | 0,071 |
| PEI-0H-10 | 0,403 | 0,067 | 0,653 | 0,105 |

Diese Daten belegen, daß diese Modifizierungen von PEI mit Tris die initiale Zelladhäsion und auch die Zellproliferation im Vergleich zu unmodifiziertem PEI erhöht. Die Modifizierung mit Tris(hydroxymethyl)-aminomethan bewirkt folglich eine Erhöhung der Zell- und Gewebeverträglichkeit von PEI-Membranen.

### Beispiel 7

In einem Faser-in-Faser-Bioreaktor mit zwei unterschiedlich großen, ineinander geschobenen Hohlfasern wurden humane C3A-Leberzellen bis zu 13 Tage lang kultiviert. Mittels dieser Anordnung entsteht ein Bioreaktor mit drei Kompartiments. Die äußere Hohlfaser bestand aus Polysulfon (PSU), die innere Hohlfaser aus unmodifizierten bzw. aus mit Amin-funktionalisierten Polyetherimid (PEI). Das äußere Kompartiment (außerhalb der äußeren Faser) wurde mit Zellkulturmedium durchströmt, im mittleren Kompartiment (Kompartiment zwischen den beiden Hohlfasern) wurden C3A-Zellen (humane Leberzellen) mit einer Dichte von 2,3 x 10⁶ Zellen/ml angesiedelt und kultiviert. Das innere Kompartiment (Lumen der inneren Faser) wurde nicht durchströmt; hier befand sich Luft. Die Besiedelung der dem mittleren Kompartiment zugewandten Oberflächen beider PEI-Hohlmembranen mit C3A-Zellen wurde mit REM-Aufnahmen dokumentiert. Gleichzeitig wurde die Zellproliferation mit einem LDH-Test verfolgt. Die Albumin-Produktion der C3A-Zellen als Nachweis für ihre Funktionalität wurde durch einen ELISA bestimmt.

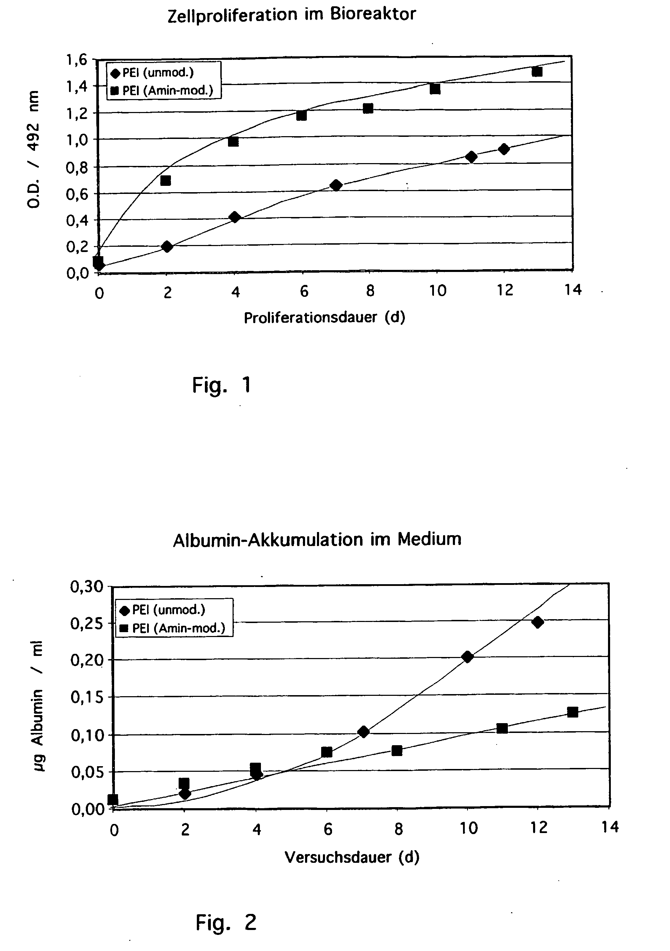

Die Proliferation von C3A-Zellen im Bioreaktor ist bei Verwendung einer mit Amin-modifizierter PEI-Innenfaser deutlich höher als mit unmodifizierter PEI-Innenfaser, wie dies im direkten Vergleich beider Innenfasern in der nachfolgenden Figur 1 dargestellt ist. Die Bedingungen für das Zellwachstum und die Zellteilung sind folglich auf der Amin-modifizierten Hohlfasermembran günstiger als auf unmodifizierten Oberfläche, was die bessere Gewebeverträglichkeit belegt.

Rasterelektronenmikroskopische Aufnahmen der zellkontaktierten Oberflächen der unmodifizierten und der Amin-funktionalisierten PEI-Hohlmembranen zeigen, daß nach ca. 2 Wochen Kultivierungsdauer beide Membranen mit einer konfluenten Schicht von C3A-Zellen bedeckt sind.

Transmissionselektronenmikroskopische Aufnahmen belegen, daß die C3A-Zellen besser an der Amin-modifizierten PEI-Innenfaser haften und auf dieser Faser Aggregate bilden und Anzeichen der Bildung einer extrazellulären Matrix. Auf der PSU-Außenfaser konnte keine Haftung nachgewiesen werden.

Während der Kultivierung im Faser-in-Faser-Bioreaktor wurde die Menge an Albumin, die von den C3A-Zellen produziert und ins Medium abgegeben wurde, als Nachweis für ihrer Funktionalität bestimmt. Die Albuminkonzentration im Medium erhöhte sich im Beobachtungszeitraum im Bioreaktor bei Verwendung sowohl der unmodifizierten PEI-Innenfasern als auch bei Verwendung der mit Aminmodifizierten PEI-Innenfasern (Figur 2). Der Vergleich mit der Zellproliferation (Figur 1) legt nahe, daß C3A-Zellen auf unmodifizierten PEI-Membranen eher ihre Funktionalität entwickeln, als ihre Vermehrung vorantreiben. Auf Amin-modifizierten Hohlfasern erfolgt eine schnelle Vermehrung mit geringerer Ausbildung an Funktionalität.

Von den nachfolgenden Zeichnungen zeigen:
- Fig. 1: Die Proliferation von C3A-Zellen auf einer unmodifizierten und einer Amin-modifizierten PEI-Hohlmembran in einem Bioreaktor mit Faser-in-Faser-Design und
- Fig. 2: die Akkumulation von Albumin im Kulturmedium in Abhängigkeit von der Kulturzeit bei Verwendung einer unmodifizierten und einer Amin-funktionalisierten PEI-Membran.

## Patentansprüche

1. Verfahren zur Herstellung einer Trägermembran eines Biohybridorgans aus einer asymmetrisch funktionalisierten porösen Polyimid-Membran, wobei als Ausgangsmaterial eine zuvor formierte poröse Polyimid-Membran eingesetzt wird, eine Oberfläche der Polyimid-Membran mit einer Funktionalisierungslösung für einen Zeitraum von 1 sec bis 1 h in Kontakt gebracht wird, die Funktionalisierungslösung mindestens einen darin zumindest teilweise gelösten Modifikator enthält, der eine Aminogruppe zur kovalenten Kopplung des Modifikators an das Polyimid sowie zusätzlich mindestens eine weitere funktionelle Gruppe pro Molekül aufweist, wobei es sich bei der weiteren funktionellen Gruppe um eine Amino-, Hydroxyl-, Carboxyl- und/oder Sulfonsäuregruppe oder um zellspezifische Liganden, die direkt oder über ein Spacermolekül an den Rest des Modifikatormoleküls gebunden sind, handelt und die weitere funktionelle Gruppe derart ausgewählt ist, dass sie nach der kovalenten Kopplung an das Polyimid und somit nach Funktionalisierung der Polyimid-Membran in freier Form vorliegt, und die Polyimid-Membran während des oder nach dem In-Kontakt-Bringen mit der Funktionalisierungslösung auf eine erhöhte Temperatur gebracht wird und anschließend gereinigt und getrocknet wird.

2. Verfahren nach Anspruch 1, **dadurch gekennzeichnet, dass** eine oder mehrere der folgenden Maßnahmen durchgeführt wird: die Polyimid-Membran wird 5 sec bis 10 min mit der Funktionalisierungslösung in Kontakt gebracht, die Polyimid-Membran wird 1 sec bis 1 h, insbesondere 5 sec bis 10 min, auf eine Temperatur von 50 bis 100°C, insbesondere von 70 bis 90°C, erhitzt, als Funktionalisierungslösung wird eine wässrige Lösung eingesetzt und die Polyimid-Membran wird zum Reinigen in an sich bekannter Weise durch Waschen, Extrahieren etc. mit Wasser behandelt.

3. Verfahren nach Anspruch 1 oder 2, **dadurch** erhältlich, dass die Konzentration der Modifikatoren in der Funktionalisierungslösung 0,1 bis 20 Gew.-%, insbesondere 1 bis 10 Gew.-%, und weiterhin insbesondere 1 bis 5 Gew.-%, beträgt.

4. Verfahren nach einem der Ansprüche 1 bis 3, **dadurch gekennzeichnet, dass** Modifikatoren mit einer primären oder sekundären Aminogruppe eingesetzt werden.

5. Verfahren nach einem der vorhergehenden Ansprüche 1 bis 4, **dadurch gekennzeichnet, dass** eine trockene Polyimid-Membran als Ausgangsmaterial eingesetzt wird.

6. Verfahren nach einem der vorhergehenden Ansprüche 1 bis 5, **dadurch gekennzeichnet, dass** Diamine, Polyamine, oder aminierte Polyethylenglykole unterschiedlicher Kettenlänge als Modifikatoren eingesetzt werden.

7. Verfahren nach einem der vorhergehenden Ansprüche 1 bis 6, **dadurch gekennzeichnet, dass** eine Ausgangsmembran mit einer schaumartigen radialkapillarfreien Struktur eingesetzt wird.

8. Verfahren nach einem der vorhergehenden Ansprüche 1 bis 7, **dadurch gekennzeichnet, dass** auch die andere Oberfläche der Polyimid-Membran mit einer weiteren Funktionalisierungslösung gemäß einem der vorherigen Ansprüche behandelt wird, die jedoch einen Modifikator oder Modifikatoren enthält, der bzw. die sich von demjenigen oder denjenigen der ersten Funktionalisierungslösung unterscheidet bzw. unterscheiden.

9. Verfahren nach einem der vorhergehenden Ansprüche 1 bis 8, **dadurch gekennzeichnet, dass** die Funktionalisierungslösung einen solchen Modifikator enthält, dass die damit behandelte Membranoberfläche hämokompatibel oder gewebekompatibel funktionalisiert ist.

10. Biohybridorgan mit einer Trägermembran, die gemäß dem Verfahren nach einem oder mehreren der Ansprüche 1 bis 9 hergestellt ist.

## Claims

1. Method for producing a carrier membrane of a biohybrid organ from an asymmetrically functionalised porous polyimide membrane, wherein a previously formed porous polyimide membrane is used as a starting material, a surface of the polyimide membrane is brought into contact with a functionalisation solution for a period of 1 sec to 1 h, the functionalisation solution contains at least one modifier at least partially dissolved therein which comprises an amino group for covalent coupling of the modifier with the polyimide and additionally at least one further functional group per molecule, wherein the further functional group is an amino, hydroxyl, carboxyl and / or sulphonic acid group or cell-specific ligands which are bonded, directly or by means of a spacer molecule, with the rest of the modifier molecule and the further functional group is selected in such a way that, after covalent coupling with the polyimide and thus after functionalisation of the polyimide membrane, it exists in a free form, and the polyimide membrane is brought, during or after bringing into contact with the functionalisation solution, to an increased temperature and subsequently purified and dried.

2. Method according to claim 1, **characterised in that** one or more of the following measures is / are implemented: the polyimide membrane is brought into contact with the functionalisation solution for 5 sec to 10 min, the polyimide membrane is heated for 1 sec to 1 h, in particular 5 sec to 10 min, to a temperature of 50 to 100°C, particularly 70 to 90°C, an aqueous solution is used as a functionalisation solution and the polyimide membrane is treated with water for purification in a way known in itself by washing, extracting, etc.

3. Method according to claim 1 or 2, obtainable in that the concentration of the modifiers in the functionalisation solution is 0.1 to 20% by weight, in particular 1 to 10% by weight and more particularly 1 to 5% by weight.

4. Method according to one of the claims 1 to 3, **characterised in that** modifiers are used with a primary or secondary amino group.

5. Method according to one of the preceding claims 1 to 4, **characterised in that** a dry polyimide membrane is used as a starting material.

6. Method according to one of the preceding claims 1 to 5, **characterised in that** diamines, polyamines or aminated polyethylene glycols of different chain length are used as modifiers.

7. Method according to one of the preceding claims 1 to 6, **characterised in that** a starting membrane is used with a foam-like radial capillary free structure.

8. Method according to one of the preceding claims 1 to 7, **characterised in that** the other surface of the polyimide membrane is also treated with a further functionalisation solution according to one of the preceding claims which contains, however, a modifier or modifiers which differs / differ from those of the first functionalisation solution.

9. Method according to one of the preceding claims 1 to 8, **characterised in that** the functionalisation solution contains such a modifier that the membrane surface treated with it is functionalised in a haemo-compatible or tissue-compatible way.

10. Biohybrid organ with a carrier membrane which is produced according to the method according to one or more of the claims 1 to 9.

## Revendications

1. Procédé de fabrication d'une membrane support d'un organe biohybride à partir d'une membrane polyimide poreuse fonctionnalisée de manière asymétrique, dans lequel une membrane polyimide poreuse préalablement formée est utilisée comme matériau de départ, une surface de la membrane polyimide est mise en contact avec une solution de fonctionnalisation pendant une durée de 1 s à 1 h, la solution de fonctionnalisation contient au moins un modificateur dissous au moins partiellement dans celle-ci, lequel comporte un groupe amino pour le couplage covalent du modificateur avec le polyimide ainsi qu'au moins un autre groupe fonctionnel par molécule, dans lequel l'autre groupe fonctionnel est un groupe amino, hydroxyle, carboxyle et/ou acide sulfonique ou des ligands cellulaires spécifiques qui sont liés au radical de la molécule de modificateur directement ou par l'intermédiaire d'une molécule d'espacement et l'autre groupe fonctionnel est choisi de telle sorte qu'il se présente sous forme libre après le couplage covalent avec le polyimide et après la fonctionnalisation résultante de la membrane polyimide, et la membrane polyimide est amenée à une température élevée pendant ou après sa mise en contact avec la solution de fonctionnalisation, puis elle est nettoyée et séchée.

2. Procédé selon la revendication 1, **caractérisé en ce qu'**une ou plusieurs des actions suivantes sont effectuées: la membrane polyimide est mise en contact de 5 s à 10 min avec la solution de fonctionnalisation, la membrane polyimide est chauffée de 1 s à 1 h et de préférence, de 5 s à 10 min, à une température de 50 à 100°C et de préférence, de 70 à 90 °C, une solution aqueuse est utilisée comme solution de fonctionnalisation et la membrane polyimide, pour son nettoyage, est traitée avec de l'eau d'une manière connue en soi, par lavage, extraction etc.

3. Procédé selon la revendication 1 ou 2, qui s'obtient avec une concentration des modificateurs dans la solution de fonctionnalisation qui va de 0,1 à 20 % en poids et en particulier, de 1 à 10 % en poids et de préférence , de 1 à 5 % en poids.

4. Procédé selon l'une quelconque des revendications 1 à 3, **caractérisé en ce que** des modificateurs ayant un groupe amino primaire ou secondaire sont utilisés.

5. Procédé selon l'une quelconque des revendications précédentes 1 à 4, **caractérisé en ce qu'**une membrane polyimide sèche est utilisée comme matériau de départ.

6. Procédé selon l'une quelconque des revendications précédentes 1 à 5, **caractérisé en ce qu'**une diamine, une polyamine ou un polyéthylène glycol aminé de différentes longueurs de chaîne sont utilisés comme modificateurs.

7. Procédé selon l'une quelconque des revendications précédentes 1 à 6, **caractérisé en ce qu'**une membrane de départ dotée d'une structure poreuse exempte de capillaires radiaux est utilisée.

8. Procédé selon l'une quelconque des revendications précédentes 1 à 7, **caractérisé en ce que** l'autre surface de la membrane polyimide est également traitée avec une autre solution de fonctionnalisation selon l'une quelconque des revendications précédentes, laquelle contient un modificateur ou plusieurs modificateurs qui diffère(nt) toutefois de celui ou ceux de la première solution de fonctionnalisation.

9. Procédé selon l'une quelconque des revendications précédentes 1 à 8, **caractérisé en ce que** la solution de fonctionnalisation contient un modificateur tel que la surface de membrane ainsi traitée est fonctionnalisée de manière à être hémocompatible ou compatible avec les tissus.

10. Organe biohybride comportant une membrane support qui est fabriquée selon le procédé selon une ou plusieurs des revendications 1 à 9.
